# EUROPEAN PATENT APPLICATION

(11) **EP 3 320 888 A1**
(43) Date of publication of application: **16.05.2018**
(21) Application number: 17198974.2
(22) Date of filing: 27.10.2017
(51) Int. Cl.: A61H 7/00

(54) **MASSAGE SYSTEM INCLUDING MASSAGE MACHINE**

(30) Priority: 15.11.2016 JP 2016222336
(71) Applicant: Family Inada Co., Ltd., Osaka 532-0004 (JP)
(72) Inventor: INADA, Nichimu, Osaka, Osaka 532-0004 (JP)
(74) Representative: Horn Kleimann Waitzhofer Patentanwälte PartG mbB

(57) **Abstract**

There is provided a massage system including a massage machine in which artificial intelligence is utilized so as to enable suitable treatment depending on a timely health condition of a treatment target person. The massage system includes a massage machine that has a treatment unit for treating the treatment target person, a control unit for controlling the treatment unit, a voice input unit, and a voice output unit, and a server that is connected to the massage machine via a communication network. The massage machine transmits voice information of the treatment target person which is input from the voice input unit, to the server via the communication network. The server causes an artificial intelligence unit to determine treatment suitable for the treatment target person, based on the voice information of the treatment target person, and transmits the treatment to the massage machine. The massage machine is configured to output the transmitted treatment from the voice output unit.

## Description

### TECHNICAL FIELD

The present invention relates to a massage system in which a massage machine and a server are connected to each other so as to enable interaction therebetween via a network.

### BACKGROUND ART

In the related art, as a massage machine configured to perform suitable massage (treatment) depending on a physical condition of a treatment target person, a massage machine is known in which diagnostic inquiries are given to the treatment target person so as to determine a physical condition of a user, based on answers of the treatment target person (for example, refer to Patent Document 1). In this massage machine, the diagnostic inquiries are given to the treatment target person, and the treatment target person answers either "Yes" or "No" to the diagnostic inquiries.

### RELATED ART DOCUMENT

### PATENT DOCUMENT

Patent Document 1: JP-A-2000-70318

### SUMMARY OF THE INVENTION

### PROBLEM THAT THE INVENTION IS TO SOLVE

However, according to the massage machine disclosed in Patent Document 1 above, the content of the diagnostic inquiries given to the treatment target person is set in advance, and the treatment target person answers either "Yes" or "No". Accordingly, the treatment content selected from the determination of the physical condition is limited. If the treatment content needs to be adjusted by more precisely determining the physical condition of the treatment target person, it is necessary to set treatment in advance according to the content of enormous diagnostic inquiries and answers, thereby causing practical difficulties.

Therefore, the present invention aims to provide a massage system including a massage machine in which artificial intelligence is utilized so as to enable suitable treatment depending on a timely health condition of a treatment target person.

### MEANS FOR SOLVING THE PROBLEM

In order to achieve the above-described object, a massage system according to the present invention includes a massage machine that has a treatment unit for treating a treatment target person, a control unit for controlling the treatment unit, a voice input unit, and a voice output unit, and a server that is connected to the massage machine via a communication network. The massage machine transmits voice information of the treatment target person which is input from the voice input unit, to the server via the communication network. The server causes an artificial intelligence unit to determine treatment suitable for the treatment target person, based on the voice information of the treatment target person, and transmits the treatment to the massage machine. The massage machine is configured to output the transmitted treatment from the voice output unit.

"The voice information" described in the specification and claims includes voice information (feelings which can be determined from accents, a voice tone, conversation speed, or a time needed to answer to a question), and utterance information (worrying sites or answer content to a question) of the treatment target person. In addition, "the treatment determination performed by the artificial intelligence unit" can include "automatic selection of a treatment course", "strength of the automatically selected treatment course", "advice on health", and "site to be intensively treated".

According to this configuration, if the treatment target person inputs the voice information to the massage machine, the voice information is transmitted to the server via the communication network, and the artificial intelligence unit in the server determines suitable treatment, based on the input voice information of the treatment target person. For example, in a case where information on "a stiff site" of the treatment target person is obtained from the voice information, it is possible to determine that a course for intensively treating the site is suitable for the treatment target person.

Then, the treatment determined by the artificial intelligence unit is notified to the treatment target person via the communication network. Accordingly, the treatment target person recognizes the treatment determined by the artificial intelligence unit, based on his or her own voice information, thereby enabling the treatment target person to determine whether or not to perform the treatment.

In addition, the massage machine may include a physical information acquisition unit that acquires physical information relating to the treatment target person. The control unit may transmit the physical information of the treatment target person which is acquired by the physical information acquisition unit, to the server via the communication network. The server may be configured to determine treatment suitable for the treatment target person by causing the artificial intelligence unit to consider the physical information of the treatment target person in addition to the voice information of the treatment target person.

"The physical information acquisition unit" described in the specification and claims includes a sphygmomanometer, a body composition meter, and a stress index measurement instrument. In addition, "the physical information" includes "pulse rate", "body weight", "body fat percentage", and "stress index". As the physical information, information obtained before the treatment target person starts treatment (massage) is transmitted.

According to this configuration, the artificial intelligence unit can comprehensively determine the treatment suitable for the treatment target person from the information in which the physical information is considered in addition to the voice information of the treatment target person.

In addition, at least any one of the massage machine and the server may include an information storage unit that stores personal information of the treatment target person.

"The personal information of the treatment target person" described in the specification and claims can include "age", "weight", "height", and "gender". The personal information can include the physical information relating to the treatment target person. "The information storage unit" can be a partial region in the storage unit of the massage machine, or a partial region in the storage unit of the server. For example, the information relating to the treatment target person is accumulated in the server, and the suitable treatment is determined by the artificial intelligence unit, based on the information and the voice information input by the treatment target person. In this manner, the artificial intelligence unit can determine the treatment suitable for the treatment target person, based on the voice information at that time and the accumulated information. If the physical information of the treatment target person is stored in the storage unit in advance, it is possible to determine the treatment suitable for the treatment target person by considering the physical information in the past and a change in the physical information at that time.

According to this configuration, the artificial intelligence unit can comprehensively determine the treatment suitable for the treatment target person from the voice information input by the treatment target person and the personal information stored in the information storage unit. For example, even if the same voice information is obtained from different treatment target persons, the treatment suitable for the treatment target person can be determined in accordance with the personal information (age or gender) of each treatment target person. In addition, it is possible to determine the treatment suitable for the treatment target person by considering an environment (time zone or season).

In addition, the artificial intelligence unit may extract information relating to the treatment of the treatment target person from the voice information of the treatment target person. The artificial intelligence unit may be configured to determine the treatment suitable for the treatment target person by considering the information relating to the treatment of the treatment target person.

"The information relating to the treatment of the treatment target person" described in the specification and claims includes extracting information of the "neck", the "shoulder", the "back", the "waist, the "leg", and the "foot", as a treatment-requiring site from the voice information. For example, the above-described site can be extracted as the treatment-requiring site from the voice information such as "My shoulder is stiff' and "My leg is tired".

According to this configuration, the artificial intelligence unit can determine the treatment suitable for the treatment-requiring site in the treatment target person at that time, from the information relating to the treatment of the treatment target person.

In addition, the artificial intelligence unit may determine feelings of the treatment target person, based on the voice information of the treatment target person, and may be configured to determine the treatment suitable for the treatment target person by considering information of the feelings. "The feelings of the treatment target person" described in the specification and claims includes feelings determined from keywords such as "hot", "cold", and "annoyed" of the voice information.

According to this configuration, the feelings can be determined from the voice information of the treatment target person, and the treatment suitable for the treatment target person can be notified to the treatment target person by considering the feelings. For example, treatment for stimulating an acupuncture point which can ease the feelings can be included in the treatment suitable for the treatment target person. The "acupuncture point" described in the specification and claims represents a so-called "pressure point".

In addition, the artificial intelligence unit may be configured to determine the treatment suitable for the treatment target person by determining activity information of the treatment target person from the voice information of the treatment target person and by considering the activity information. "The activity information of the treatment target person" described in the specification and claims represents information on that day (what kind of life activities), and can include "all day, standing work", "all day, outside work", and "all day, desk work".

According to this configuration, the activity information of the treatment target person is considered so as to determine a type or a site of the treatment. In this manner, it is possible to notify the treatment target person of the treatment suitable for the treatment target person. For example, in a case where the treatment target person carries out the standing work and the outside work, the treatment target person can be recommended to receive the intensive treatment for the legs, or in a case where the treatment target person carries out desk work, the treatment target person can be recommended to receive intensive treatment for the waist.

In addition, the artificial intelligence unit may determine information relating to a body of the treatment target person from the voice information of the treatment target person, and may be configured to determine the treatment suitable for the treatment target person by considering the information relating to the body. "The information relating to the body of the treatment target person" described in the specification and claims includes information (keywords) such as "I have a backache", "My shoulder is stiff', and "I am in a slightly bad condition". In a case where the physical condition of the treatment target person is stored, even if the information (keywords) obtained from the voice information on the day is the same as that obtained the previous day, it is possible to determine that the treatment target person needs the treatment which is different from that of the previous day, based on the information relating to the body on the day. In addition, it is possible to change the question content, the answer content, and the answer action (treatment) which are given from the artificial intelligence unit to the treatment target person.

According to this configuration, depending on a state of the body of the treatment target person, the content of the treatment can be determined. For example, in a case of "I have a backache", the treatment target person feels back pain. Accordingly, the treatment strength for the waist can be weakened. In a case of "My shoulder is stiff', the treatment target person can be recommended to receive an intensive shoulder treatment course.

In addition, the massage machine may include an imaging unit that images a face of the treatment target person. The artificial intelligence unit may perform determination based on a Visual diagnosis method from the face of the treatment target person which is imaged by the imaging unit, and is configured to determine the treatment suitable for the treatment target person by considering the determination based on the Visual diagnosis method in addition to the determination based on the voice information of the treatment target person. As "the imaging unit" described in the specification and claims, it is possible to utilize those which can capture an image so that facial expressions or facial colors of the treatment target person can be recognized, such as a camera. The Visual diagnosis method is a diagnostic method for diagnosing a health condition by observing the body or the face.

According to this configuration, based on the imaging data of the face of the treatment target person, the treatment suitable for the treatment target person can be determined by considering the diagnosis using the Visual diagnosis method (diagnosing feelings or the health condition from facial expressions and facial colors).

In addition, at least any one of the massage machine and the server may cause a history storage unit to store a usage history including the treatment determined based on the voice information of the treatment target person. The artificial intelligence unit may be configured to determine the treatment suitable for the treatment target person by considering the usage history stored in the history storage unit in addition to new voice information of the treatment target person. "The usage history" described in the specification and claims can include "used date and time", "used period of time", "used time zone", "used course", and "physical condition".

According to this configuration, the determination using the artificial intelligence unit can be made by considering the usage history including the determination in the past and the treatment selected by the treatment target person.

In addition, the artificial intelligence unit may ask the treatment target person a question or may answer a question from the treatment target person. In a case where the artificial intelligence unit obtains an answer to the question from the treatment target person, the artificial intelligence unit may be configured to determine the treatment suitable for the treatment target person from a plurality of answers by obtaining a new answer from the treatment target person after asking a question relating to the obtained answer. "The question to the treatment target person from the artificial intelligence unit or the answer to the question from the treatment target person" described in the specification and claims includes conversational questions and answers which are exchanged multiple times. For example, in a case where the artificial intelligence unit asks the treatment target person a question, the artificial intelligence unit can ask the treatment target person the following questions, for example, such as "Do you have any painful site on your body recently?", "Do you have any stiff site?", "How is your physical condition today?", "Do you feel tired?", and "How do you feel today?". Then, in a case where the treatment target person answers to the questions, for example, in a case where the answer is "I have a backache", the treatment target person feels back pain. Accordingly, the treatment strength for the waist can be weakened. In a case where the answer is "My shoulder is stiff', the treatment target person can be recommended to receive an intensive shoulder treatment course. In a case where the answer is "I am in a slightly bad condition", it is possible to confirm detailed content of the bad condition. In addition, in a case where no answer is heard, it is possible to ask the question again. If the meaning of the answer content cannot be recognized, it is possible to ask the meaning again, such as "What do you mean?" In a case where the artificial intelligence unit asks the treatment target person a question, it is possible to guide the answer content of the treatment target person so as to hear information for determining the suitable treatment.

On the other hand, for example, in a case where the treatment target person answers to the questions from the artificial intelligence unit, the treatment target person can answer to the questions (utterances) from the artificial intelligence unit, such as "I feel tired on my leg today", "I think my back is tired today", and "My shoulder is stiff today". Then, for example, in a case of the answer such as "I feel tired on my leg today", the artificial intelligence unit can respond to the answer, such as "Then, you are recommended to receive an intensive leg treatment course. What's your opinion?" In a case of an ambiguous answer such as "I think my back is tired today", in order to obtain further detailed information, the artificial intelligence unit can ask the treatment target person a question such as "How do you feel your back now?" In this manner, the detailed content can be heard from the treatment target person.

According to this configuration, the conversation relating to the treatment can be exchanged between the treatment target person and the artificial intelligence unit. The artificial intelligence unit extracts and collects the information relating to the treatment from the content of the conversation exchanged between the treatment target person and the artificial intelligence unit. In this manner, the treatment suitable for the treatment target person at that time can be determined so as to notify the treatment target person of the suitable treatment.

In addition, the artificial intelligence unit may exchange a casual conversation with the treatment target person, and may be configured to determine the treatment suitable for the treatment target person from the content of the conversation. "The casual conversation" described in the specification and claims can include the conversation such as the casual conversation about the news of the day, the weather of the day, and memorized family members, or chatting. For example, the casual conversation is exchanged between the artificial intelligence unit and the treatment target person, thereby enabling the treatment target person to relaxedly receive the treatment. For example, the content of the conversation can include "It's hot today", or "It's raining today". Then, the artificial intelligence unit can determine the treatment suitable for the treatment target person at that time by determining the health condition of the treatment target person from the content of the conversation with the treatment target person or the voice tone of the treatment target person.

According to this configuration, the artificial intelligence unit can notify the treatment target person of the suitable treatment by properly determining the treatment suitable for the treatment target person at that time from the content of the conversation exchanged between the treatment target person and the artificial intelligence unit. In addition, the conversation such as chatting is exchanged therebetween during the treatment, thereby enabling the treatment target person to feel as if he or she is treated by a massage master. Accordingly, the treatment target person can more relaxedly receive the treatment.

In addition, the artificial intelligence unit may confirm the treatment of the massage machine from the voice output unit while the treatment target person is treated. The artificial intelligence unit may determine whether or not to change an operation of the massage machine, based on the answer of the treatment target person which is input from the voice input unit. In a case where the artificial intelligence unit determines that the operation of the massage machine needs to be changed, the artificial intelligence unit may be configured to notify the treatment target person of the determined change result from the voice output unit of the massage machine. "The confirmation of the treatment of the massage machine from the voice output unit while the treatment target person is treated" and "the change of the operation of the massage machine" described in the specification and claims include the following cases, for example. The artificial intelligence unit asks the treatment target person a question for reconfirmation, such as "Doesn't massage hurt your back?" If there is an answer such as "It hurts me a little bit" from the treatment target person, a signal indicating a change result of weakening the treatment of the waist is transmitted to the massage machine, thereby performing an operation for weakening the treatment of the waist. In addition, for example, the case includes reconfirmation such as "How do you feel about shoulder massage?" and "How about the strength?", and adjusting the strength of the shoulder treatment based on the answer of the question. In addition, the case includes a change in the operation of the massage machine in response to the answer of the treatment target person after the suitable treatment can be confirmed by the conversation exchanged between the artificial intelligence unit and the treatment target person. The change in the operation of the massage machine can start, based on the conversation such as "It hurts me a little bit" from the treatment target person side. In a case where the confirmation such as "Good feeling" is obtained, the treatment is continued without any change. This operation is included in the above-described case.

According to this configuration, based on the content of the conversation exchanged between the treatment target person and the artificial intelligence unit during the treatment, the artificial intelligence unit can properly determine the treatment suitable for the treatment target person during the treatment, and can determine whether or not to change the treatment operation.

In addition, the massage machine may be a chair-type massage machine that has at least a seat unit on which the treatment target person sits, and a backrest unit that supports an upper body of the treatment target person sitting on the seat unit from behind. The control unit may be configured to adjust an angle of at least the backrest unit to a suitable angle, based on the determination of the artificial intelligence unit.

According to this configuration, in the chair-type massage machine, based on the determination of the artificial intelligence unit, the control unit adjusts the angle of the backrest unit, thereby enabling the treatment target person to receive the suitable treatment. For example, in a case where the treatment target person has a poor health condition or feels uncomfortable, it is possible to reduce the angle of tilting the backrest unit. In addition to the angle adjustment of the backrest unit, the respective treatment units can be additionally adjusted.

### ADVANTAGE OF THE INVENTION

According to the present invention, the artificial intelligence unit determines the health condition of the treatment target person, based on the voice information of the treatment target person, and can notify the treatment target person of the suitable treatment by determining the treatment suitable for the treatment target person at that time. Therefore, the treatment target person can receive the suitable treatment depending on the health condition at that time.

### BRIEF DESRIPTION OF THE DRAWINGS

Fig. 1 is a block diagram illustrating a configuration of a massage system including a massage machine according to an embodiment of the present invention.
Fig. 2 is an overall configuration diagram of the massage system including the massage machine illustrated in Fig. 1.
Fig. 3 is a perspective view of the massage machine illustrated in Fig. 2.
Fig. 4 is a functional block diagram of the massage machine illustrated in Fig. 3.

### BEST MODE FOR CARRYING OUT THE INVENTION

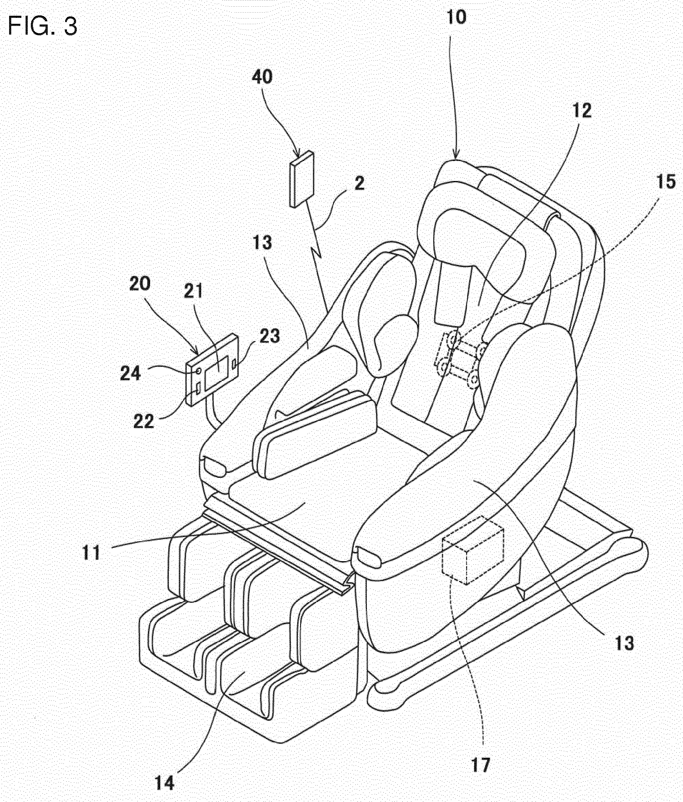

Hereinafter, an embodiment according to the present invention will be described with reference to the drawings. In the following embodiment, an example will be described in which a massage machine 10 and a cloud-type server 50 are connected to each other via a communication network 3. It is assumed that the concept of back, forth, right, and left directions in the specification and claims coincides with the concept of the directions which are viewed from a treatment target person when the treatment target person sits on a seat unit 11 of the massage machine 10 illustrated in Fig. 3.

### Configuration of Massage System

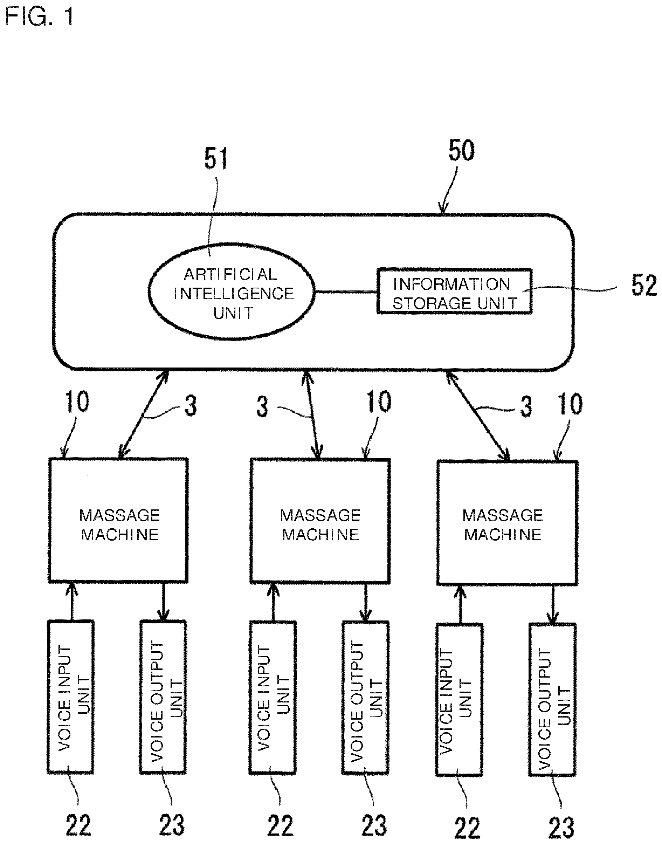

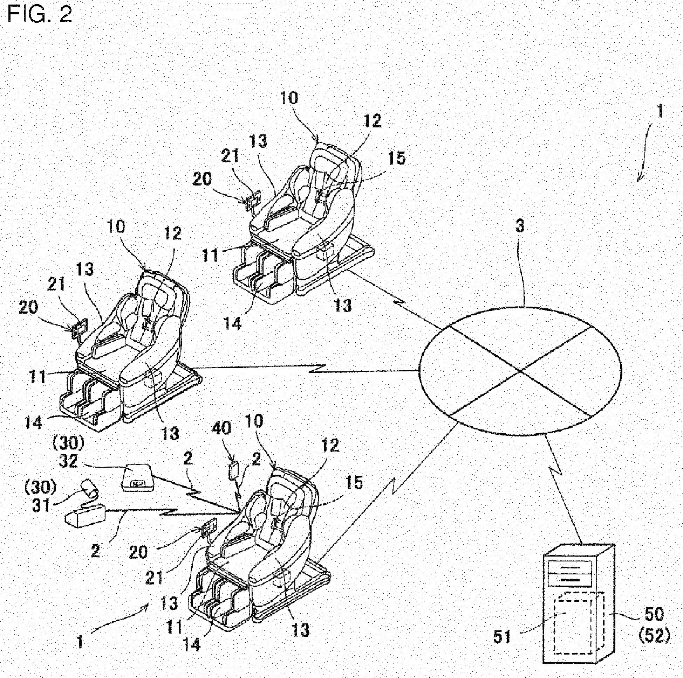

Fig. 1 is a block diagram illustrating a configuration of a massage system 1 including the massage machine 10 according to an embodiment, and Fig. 2 is an overall configuration diagram of the massage system 1 including the massage machine 10 illustrated in Fig. 1. The massage system 1 including the massage machine 10 according to the present embodiment includes a plurality (three) of the massage machines 10 for treating the treatment target person, the communication network 3, and the server 50. The server 50 is connected to the massage machine 10 so as to enable communication therebetween via the communication network 3.

The above-described massage machine 10 has the seat unit 11 on which the treatment target person sits, and a backrest unit 12 for supporting an upper body of the treatment target person sitting on the seat unit 11 from behind. The seat unit 11 has an arm rest 13 at right and left positions, and has a foot rest 14 in a front portion of the seat unit 11. In this example, only a treatment unit (roller unit) 15 of the backrest unit 12 is illustrated. A treatment unit such as an air bag (not illustrated) is included in other places. In addition, an operation device 20 for operating the massage machine 10 is included in the place of the arm rest 13 on the right side. The operation device 20 according to the present embodiment is a tablet-type operation device, and has a screen 21 operated by the treatment target person, a voice input unit (microphone) 22, and a voice output unit (speaker) 23.

Furthermore, the massage machine 10 according to the present embodiment includes a terminal 40 in addition to the above-described operation device 20. As the terminal 40, it is possible to use a terminal such as a smartphone and a tablet. It is possible to use those which include the voice input unit (microphone) 22 and the voice output unit (speaker) 23. The terminal 40 can communicate with the massage machine 10 via wireless communication (Bluetooth (registered trademark)) 2. The terminal 40 may be connected to the massage machine 10 by using other forms of wireless or wired communication.

In addition, the massage machine 10 according to the present embodiment includes a measurement unit 30 as a physical information acquisition unit which obtains physical information relating to the treatment target person. As the measurement unit 30, the massage machine 10 includes a sphygmomanometer 31 and a body composition meter 32 which are configured to be separate from the massage machine 10. The sphygmomanometer 31 and the body composition meter 32 can communicate with the massage machine 10 through the wireless communication (Bluetooth (registered trademark)) 2. The measurement unit 30 may be connected to the massage machine 10 by using other forms of wireless or wired communication. In addition, the measurement unit 30 may be incorporated in the massage machine 10. In Fig. 2, the terminal 40, the sphygmomanometer 31, and the body composition meter 32 are illustrated in one of the massage machines 10. However, all of the massage machines 10 can include these configurations.

Then, the above-described server 50 is provided with an artificial intelligence unit 51. The artificial intelligence unit 51 is configured to include a computer provided with artificial intelligence. The artificial intelligence unit 51 has a function to understand utterance content of from voice information of the treatment target person ("the voice information" described in the specification and claims includes "utterance information", and the "utterance information" includes "the utterance content"). The understanding of the utterance content by the artificial intelligence unit 51 includes understanding of natural languages. The artificial intelligence unit 51 has a learning function to accumulate information understood from the utterance content in the information storage unit 52 and to utilize the information when the artificial intelligence unit 51 performs new determination.

In addition, according to the present embodiment, it is possible to utilize information of the utterance content obtained from three massage machines 10. The artificial intelligence unit 51 can utilize big data accumulated in the server 50.

### Configuration of Massage Machine

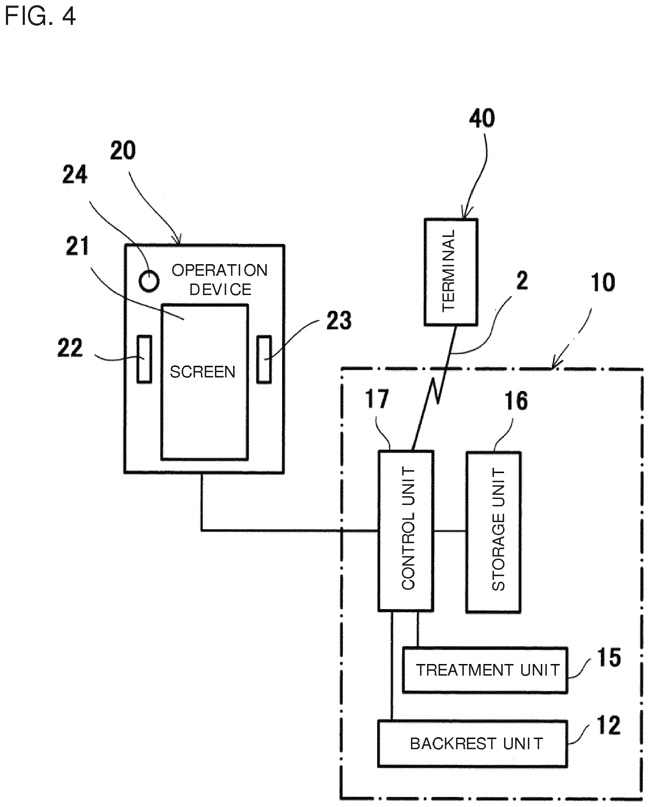

Fig. 3 is a perspective view of the massage machine 10 illustrated in Fig. 2. Fig. 4 is a functional block diagram of the massage machine 10 illustrated in Fig. 3. As illustrated in Fig. 3, the massage machine 10 includes a control unit 17 for controlling the treatment unit such as the seat unit 11, the backrest unit 12, the arm rest 13, and the foot rest 14. The control unit 17 can operate a plurality of treatment courses by combining the respective treatment units (including not only the treatment unit 15 but also an air bag (not illustrated)) with each other. The control unit 17 can change the content of the treatment course. In addition to the treatment course, the control unit 17 can operate only a portion of the treatment content. The control unit 17 controls strength of an operation performed by the treatment unit 15 and a tilting angle of the backrest unit 12.

As illustrated in Fig. 4, the control unit 17 is connected to a storage unit 16 which stores a plurality of the treatment courses having mutually different treatment content used by the treatment unit 15. The storage unit 16 can also function as any one or both of an information storage unit 52 and a history storage unit (to be described later). For example, the control unit 17 has a CPU, a ROM, and a RAM. For example, as the storage unit 16, a flash memory or a hard disk drive can be used. In addition, the operation device 20 according to the present embodiment is provided with the voice input unit 22, the voice output unit 23, and a camera 24 serving as an imaging unit. The camera 24 may include the terminal 40.

### Determination of Basic Treatment in Massage System

Hereinafter, determination of the treatment using the above-described massage system 1 will be described. In the following description, the above-described reference numerals will be given to the above-described configurations.

According to the above-described massage system 1, as described below, the conversation is exchanged between the treatment target person and the artificial intelligence unit 51, thereby causing the artificial intelligence unit 51 to determine the treatment suitable for the treatment target person. In the following example, an example in which the treatment target person first starts the conversation will be mainly described. However, the treatment target person turns on power of the massage machine 10, and the control unit 17 is connected to the artificial intelligence unit 51 via the communication network 3. In this way, the artificial intelligence unit 51 may first start the conversation.

If the treatment target person talks (inputs a voice) to the voice input unit 22 of the massage machine 10, the voice information is transmitted to the server 50 via the communication network 3. The artificial intelligence unit 51 of the server 50 understands the utterance content from the voice information of the treatment target person. The utterance content can be understood by the artificial intelligence unit 51 together with the voice information (feelings which can be determined from accents, a voice tone, or conversation speed), and the utterance information (concerns or the content relating to the treatment) of the treatment target person.

Then, the artificial intelligence unit 51 determines the treatment suitable for the treatment target person, based on the voice information. The treatment suitable for the treatment target person is treatment suitable for the health condition, the physical information, o the feelings of the treatment target person. The suitable treatment includes the determination of the treatment course suitable for the treatment target person at that time in the massage machine 10, and the determination in a state where the treatment strength of the treatment unit 15 and the angle of the backrest unit 12 are suitably arranged. As the determination of the treatment suitable for the treatment target person, the artificial intelligence unit 51 extracts a plurality of keywords from the content of the conversation with the treatment target person and the utterance content of the treatment target person. In this manner, the treatment course suitable for the treatment target person can be determined. For example, in a case where the information such as "stiff site" of the treatment target person is obtained from the voice information, it is possible to determine that the treatment course mainly for the site is suitable for the treatment target person. In a case where the information such as the treatment target person is "tired" from the voice information, it is possible to determine that the treatment course for weakening treatment is suitable for the treatment target person. In the determination of the artificial intelligence unit 51, the artificial intelligence unit 51 extracts a plurality of the keywords from the content of the conversation, and the answer may vary depending on the content, in some cases. "The determination of the artificial intelligence unit" can include "automatically selected course", "strength of the automatically selected course", "advice on health", and "site to be intensively treated".

In this way, according to the above-described massage system 1, if the treatment target person inputs the voice information to the massage machine 10, the voice information is transmitted to the server 50 via the communication network 3. In the server 50, the treatment suitable for the treatment target person is determined by the artificial intelligence unit 51.

Then, the artificial intelligence unit 51 transmits the determined treatment course to the massage machine 10 via the communication network 3. The massage machine 10 outputs the determination result such as the treatment course transmitted from the artificial intelligence unit 51 from the voice output unit 23, and notifies the treatment target person of the determination result. Based on the voice information input by himself or herself, the treatment target person recognizes the treatment course determined by the artificial intelligence unit 51, and can determine whether or not to receive the treatment course.

In addition, the treatment target person can vocally respond to the recognized determination of the artificial intelligence unit 51. In this manner, the treatment target person can vocally conversate with the artificial intelligence unit 51 with regard to the treatment. That is, the treatment target person can conversate with the artificial intelligence unit 51 with regard to the treatment as if he or she talks with a person. Based on the information obtained from the conversation, the artificial intelligence unit 51 determines the treatment suitable for the treatment target person, and can notify the treatment target person of the suitable treatment.

Furthermore, in a case where the massage system 1 is provided with the chair-type massage machine 10 (embodiment illustrated in Fig. 2), the artificial intelligence unit 51 determines the health condition of the treatment target person, and can transmits an angle signal for tilting the backrest unit 12 to the chair-type massage machine 10 in response to the physical condition of the treatment target person. Then, the control unit 17 of the massage machine 10 adjusts the angle of the backrest unit 12 to a suitable angle, based on the signal from the artificial intelligence unit 51. That is, for example, in a case where the artificial intelligence unit 51 determines the health condition of the treatment target person as described later and determines that the physical condition is not good, a signal for decreasing the angle for tilting the backrest unit 12 can be transmitted to the massage machine. Therefore, depending on the physical condition of the treatment target person, the control unit 17 adjusts the angle of the backrest unit 12, based on the determination of the artificial intelligence unit 51, and the treatment target person can receive the suitable treatment. In this example, the angle adjustment of the backrest unit 12 has been described. Alternatively, a control signal is transmitted from the artificial intelligence unit 51 to the control unit 17. In this manner, it is possible to adjust the strength or the treatment time of the respective treatment units.

### Determination Example of Another Treatment in Massage System

In the massage system 1 according to the above-described embodiment, as illustrated in Fig. 2, the sphygmomanometer 31 and the body composition meter 32 are connected to the massage machine 10 as the measurement unit 30. Therefore, the massage machine 10 obtains the physical information such as "blood pressure value", "pulse rate", "weight", and "body fat rate" of the treatment target person from the measurement unit 30, and the massage machine 10 can transmit the physical information to the server 50 via the communication network 3. The physical information is transmitted before the treatment target person is treated. The physical information relating to the treatment target person can be previously stored in the information storage unit 52 of the server 50. In addition, if there is provided a stress index measurement instrument serving as the physical information acquisition unit, it is possible to obtain the "the stress index" of the treatment target person at that time. In this way, the artificial intelligence unit 51 can comprehensively determine the treatment suitable for the treatment target person by considering the physical information of the treatment target person in addition to the voice information of the treatment target person.

In addition, at least one of the storage unit 16 of the massage machine 10 and the information storage unit 52 of the server 50 can previously store each personal information relating to the treatment target persons who utilize the respective massage machines 10. The personal information can include "age", "weight", "height", and "gender" of each treatment target person. The personal information may be stored in at least either the massage machine 10 or the server 50. The information relating to the treatment target person is stored in the storage unit 16 or the information storage unit 52. In this manner, when the artificial intelligence unit 51 determines the treatment suitable for the treatment target person, the artificial intelligence unit 51 can determine the treatment suitable for the treatment target person, based on the voice information of the treatment target person at that time and the stored information.

For example, in a case where the artificial intelligence unit 51 obtains the information indicating that the treatment target person is an elderly person, based on the information of "age", the artificial intelligence unit 51 can ask the treatment target person a question such as "Do you want to weaken the treatment strength?" Depending on the age of the treatment target person, the artificial intelligence unit 51 can adjust the strength of the treatment course, such as usual treatment intensity and weak treatment strength. In addition, the artificial intelligence unit 51 can give an advice on health corresponding to the age. In addition, the artificial intelligence unit 51 can ask the treatment target person questions, based on the information of "weight", "height", and "gender".

In this way, based on the previously stored (registered) personal information and the voice information input by the treatment target person, the artificial intelligence unit 51 comprehensively determines the treatment suitable for the treatment target person, and can adjust the treatment operation to have proper content. Therefore, for example, even if the same voice information is obtained from different treatment target persons, the artificial intelligence unit 51 can determine the suitable treatment by considering the personal information (age, gender, or usage history) of each treatment target person. In addition, it is also possible to determine the suitable treatment by considering an environment (time zone or season). Therefore, if the treatment target persons utilize the massage machine 10 at mutually different times, even if the artificial intelligence unit 51 obtains similar keywords from the voice information, the artificial intelligence unit 51 differently determines the treatment suitable for the treatment target person.

### Example of Factors for Determining Treatment Suitable for Treatment Target Person

Hereinafter, an example of factors for the artificial intelligence unit 51 to determine the treatment suitable for the treatment target person will be described. These factors serve as the information relating to the treatment of the treatment target person.

### <Extraction of Treatment Information relating to Treatment Target Person>

The artificial intelligence unit 51 can extract information relating to the treatment suitable for the treatment target person at that time, based on the voice information of the treatment target person. As the information relating to the treatment of the treatment target person, the information such as "neck", "shoulder", "back", "waist", "leg", and "foot" can be extracted from on the utterance content as treatment-requiring sites. For example, in a case where the information indicating that "My waist is tired" is extracted from the utterance content of the treatment target person, the artificial intelligence unit 51 determines that the intensive treatment course for the waist is suitable for the treatment target person, and can output a voice such as "You are recommended to receive the intensive treatment course for the waist". In a case where the information indicating that "My shoulder is stiff' is extracted, it is possible to determine that the intensive treatment course for the shoulder is suitable for the treatment target person. In addition, in a case where the information indicating that "I have a backache" is extracted, the treatment target person feels back pain. Accordingly, it is possible to determine that the treatment strength for the waist needs to be weakened. In a case where the information indicating "I am in a slightly bad condition" is extracted, the artificial intelligence unit 51 confirms detailed content such as how is the bad condition, and can determine the treatment suitable for the treatment target person, based on the detailed information. In this way, the artificial intelligence unit 51 extracts the treatment-requiring site of the treatment target person at that time, and can determine the treatment suitable for the site.

### <Extraction of Feelings from Voice Information>

Based on the voice information of the treatment target person, the artificial intelligence unit 51 can determine the feelings of the treatment target person. For example, the feelings can be determined by extracting keywords such as "hot", "cold", or "annoyed" from the utterance content of the treatment target person. In addition, the feelings of the treatment target person can be determined from accents, voice tones, or conversation speed of the treatment target person. Depending on the determined feelings of the treatment target person, the artificial intelligence unit 51 can determine the treatment suitable for the treatment target person by considering the information of the feelings. For example, in a case where the keyword such as "annoyed" is extracted, it is possible to recommend the treatment for stimulating an acupuncture point which can ease the feelings. In addition, it is possible to recommend the treatment target person to change the treatment intensity.

### <Extraction of Activity Information>

The artificial intelligence unit 51 can determine activity information of the treatment target person, based on the voice information of the treatment target person. For example, based on the utterance content, the artificial intelligence unit 51 can determine the activity information indicating what kind of life the treatment target person has on that day. For example, the activity information such as "all day, standing work", "all day, outside work", and "all day, desk work" can be extracted. Then, the artificial intelligence unit 51 can determine the treatment suitable for the treatment target person by considering the activity information of the treatment target person in addition to the determination including a type or a site of the treatment. For example, in a case where the treatment target person carries out the standing work all day, the artificial intelligence unit 51 can determine that the legs are tired, and can recommend the treatment target person to receive the intensive treatment for the legs. In addition, in a case where the treatment target person carries out the outside work, the artificial intelligence unit 51 can determine that the legs and feet are tired, and can recommend the treatment target person to receive the intensive treatment for the legs and feet. In addition, in a case where the treatment target person carries out the desk work all day, the artificial intelligence unit 51 can determine that the waist and back are tired, and can recommend the treatment target person to receive the intensive treatment for the waist and back or the treatment for stimulating an acupuncture point which is effective for the tired waist and back.

### <Extraction of Physical Information>

Based on the voice information of the treatment target person, the artificial intelligence unit 51 can determine information relating to the body of the treatment target person. The information relating to the body of the treatment target person can be determined by the artificial intelligence unit 51 extracting the information (keyword) such as "I have a backache", "My shoulder is stiff', and "I am in a slightly bad condition". Then, the information relating to the body of the treatment target person is extracted. In this manner, the artificial intelligence unit 51 can determine the suitable treatment depending on a condition of the body of the treatment target person at that time. In addition, in a case where the physical condition of the treatment target person is stored, even if the information (keyword) obtained from the voice information on the day is the same as that of the previous day, it is possible to determine the treatment which is different from that of the previous day, based on the information relating to the body of the day. In addition, it is possible to change the question content, the answer content, and the answer action (treatment) which are given from the artificial intelligence unit 51 to the treatment target person. For example, in a case where "I have a backache", the treatment target person feels back pain. Accordingly, it is possible to recommend the treatment target person to weaken the treatment strength of the waist. In a case where "My shoulder is stiff', it is possible to recommend the treatment target person to receive the intensive treatment course for the shoulder.

### <Diagnosis based on Visual Diagnosis Method>

In the massage machine 10, the operation device 20 is provided with the camera 24 for imaging the face of the treatment target person. Imaging data of the face of the treatment target person imaged by the camera 24 is transmitted from the control unit 17 to the server 50. Then, the imaging data of the face of the treatment target person which is transmitted to the server 50 is used by the artificial intelligence unit 51 so as to determine the feelings or the health condition from facial expressions and facial colors, based on a visual diagnosis method. In this manner, the artificial intelligence unit 51 can determine the treatment suitable for the treatment target person by considering the determination result of the visual diagnosis method based on the imaging data of the face of the treatment target person.

### <Utilization of Usage History>

The server 50 can cause the information storage unit 52 to previously store a usage history including the treatment determined based on the voice information of the treatment target person. In the present embodiment, the information storage unit 52 also functions as a history storage unit. The usage history stored in the information storage unit 52 can include "used date and time", "used period of time", "used time zone", "used course", and "physical condition".

Then, the artificial intelligence unit 51 can determine the treatment suitable for the treatment target person by considering the usage history stored in the information storage unit 52 in addition to the new voice information of the treatment target person. For example, the artificial intelligence unit 51 can change the content of the questions using the voice output from the physical condition of the previous day to the physical condition of the day by previously storing the physical condition of the treatment target person of the previous day. Even if the keywords extracted from the voice information of the treatment target person are the same as each other, it is possible to change the answer content or the answer action. For example, even if a keyword such as "Tilt the backrest unit" is recognized from the voice information of the treatment target person, an angle for tilting the backrest unit 12 in a previous state can be changed so as to be different from the previous angle. In addition, in a case where there is an interval on the used days of the treatment target person, the artificial intelligence unit 51 can start with the conversation such as "You have not used the massage machine for a while". Depending on the used time zone, the artificial intelligence unit 51 can start with the conversation such as "You use the massage machine earlier today".

If the usage history of the treatment target person is previously stored in this way, the artificial intelligence unit 51 can perform the conversation or the determination by considering the usage history including the determination or the selected treatment in the past. In this manner, it is possible to consider preferences corresponding to the treatment target person. In addition, since the artificial intelligence unit 51 starts with the conversation based on the usage history, the treatment target person can confirm the history in the past.

### <Extraction of Information relating to Treatment from Questions and Answers>

The artificial intelligence unit 51 can ask the treatment target person questions or can answer to questions from the treatment target person. In a case where the artificial intelligence unit 51 obtains the answer to the question from the treatment target person, the artificial intelligence unit 51 can continue the conversation so as to obtain a new answer by asking the treatment target person a question relating to the answer. Then, the artificial intelligence unit 51 can determine the treatment suitable for the treatment target person, based on a plurality of answers obtained from the treatment target person.

The questions from the artificial intelligence unit to the treatment target person or the answers to the questions from the treatment target person include conversations exchanged multiple times. For example, in a case where the artificial intelligence unit 51 asks the treatment target person the questions, for example, the artificial intelligence unit 51 can ask the treatment target person the questions such as "Do you have any painful site on your body recently?", "Do you have any stiff site?", "How is your physical condition today?", "Do you feel tired?", and "How do you feel today?". Then, in a case where an answer is obtained from the treatment target person, for example, in a case where an answer such as "I have a backache" is obtained, the treatment target person feels back pain. Accordingly, it is possible to recommend the treatment target person to weaken the treatment strength of the waist. In a case where an answer such as "My shoulder is stiff' is obtained, it is possible to recommend the treatment target person to receive the intensive treatment course for the shoulder. In a case where an answer such as "I am in a slightly bad condition" is obtained, it is possible to confirm detailed content indicating why the treatment target person has the bad condition. In addition, in a case where the answer cannot be understood (heard), it is possible to ask the treatment target person to answer to the question one more time. If the content of the answer cannot be recognized, it is possible to ask the meaning again, such as "What do you mean?" If it is possible to recognize the meaning of the utterance content of the treatment target person after hearing the answer again, it is possible to learn the meaning. Then, it is possible to determine the treatment suitable for the treatment target person from the answer by confirming the detailed content from the answer heard again. The artificial intelligence unit 51 asks the treatment target person the question. In this manner, it is possible to guide the answer content of the treatment target person so as to hear the information for determining the treatment suitable for the treatment target person at that time.

On the other hand, in a case where the treatment target person side answers to the question, the treatment target person can answer to the question (utterance) such as "My legs are tired today", "I think my back is tired today", "My shoulder is stiff today", for example. Then, for example, in a case of the answer such as "My legs are tired today", the artificial intelligence unit 51 can reply to the answer, such as "You are recommended to receive the intensive leg treatment course for intensively treating your legs. What's your opinion?" In a case of an ambiguous answer such as "I think my back is tired today", in order to obtain further detailed information, the artificial intelligence unit 51 can ask the treatment target person a question such as "How do you feel your back now?"

The questions and answers are repeatedly exchanged between the treatment target person and the artificial intelligence unit 51 in this way. Accordingly, the artificial intelligence unit 51 can extract and collect the information relating to the treatment of the treatment target person from the content of the conversation. Based on the information, it is possible to notify the treatment target person of the suitable treatment by properly determining the treatment suitable for the treatment target person at that time.

### <Extraction of Information relating to Treatment from Casual Conversation>

The artificial intelligence unit 51 exchanges a casual conversation with the treatment target person, and can determine the treatment suitable for the treatment target person, based on the content of the conversation. The casual conversation from the artificial intelligence unit 51 can include conversations such as the casual conversation about the news of the day, the weather of the day, and memorized family members, or chatting. In addition, the casual conversation is exchanged between the artificial intelligence unit 51 and the treatment target person. In this manner, the treatment target person is not bored with the conversation, and can relaxedly receive the treatment. For example, the content of the conversation can include "It was hot today" and "It will rain today, and the rainfall probability is xx%". This casual conversation is included therein. Accordingly, the treatment target person can exchange a natural conversation with the artificial intelligence unit 51. The treatment target person is not bored with the conversation with the artificial intelligence unit 51, and can relaxedly receive the treatment.

Then, the artificial intelligence unit 51 properly determines the treatment suitable for the treatment target person at that time, based on the content of the conversation with the treatment target person and the voice tone of the treatment target person, and can notify the treatment target person of the suitable treatment. In addition, the conversation is exchanged therebetween during treatment, thereby enabling the treatment target person to feel as if he or she is treated by a massage master. Accordingly, the treatment target person can relaxedly receive the treatment.

### <Change of Treatment Operation from Conversation during Treatment>

The artificial intelligence unit 51 can confirm the treatment of the massage machine 10 from the voice output unit 23 while the treatment target person is treated. Then, the artificial intelligence unit 51 obtains the answer of the treatment target person from the voice input unit 22, and can determine whether or not to change the operation of the massage machine 10, based on the obtained answer. In a case where the artificial intelligence unit 51 determines to change the operation of the massage machine 10, the artificial intelligence unit 51 can notify the treatment target person of the determination result through the voice output unit 23 of the massage machine 10. Then, in a case where the treatment target person accepts the change, a control signal for changing the operation is transmitted to the control unit 17, and the operation of the treatment unit can be changed by the control unit 17.

For example, if the artificial intelligence unit 51 asks the treatment target person a confirmation question such as "Doesn't massage hurt your back?", and if there is an answer such as "It hurts me a little bit" from the treatment target person, the artificial intelligence unit 51 asks the treatment target person a question such as "Do you want to slightly weaken the waist massage?" Then, if an answer such as "Please weaken the massage" is obtained from the treatment target person, the artificial intelligence unit 51 can transmit a change signal for weakening the waist treatment to the massage machine 10. Then, the control unit 17 causes the treatment unit to weaken the operation performed on the waist. In a case where the confirmation such as "Good feeling" is obtained, the treatment is continued without any change. In addition, the artificial intelligence unit 51 asks the confirmation questions such as "How do you feel about shoulder massage?" and "How about the strength?" For example, if the answer is "A little strong", a voice such as "The strength will be weakened a little" is output, and the signal indicating the change result of weakening the shoulder treatment is transmitted to the massage machine 10. The treatment unit is caused to weaken the shoulder treatment by the control unit 17. In addition, the treatment can be confirmed by the conversation between the artificial intelligence unit 51 and the treatment target person. The operation of the treatment unit can be changed in response to the answer of the treatment target person. In order to change the operation of the massage machine 10, the conversation can start from the treatment target person side.

In addition, after the treatment operation is changed, it is possible to ask a condition of the treatment operation again. In a case where the answer is "Still hurting" as a result of the reconfirmation, a signal for further weakening the treatment performed by the treatment unit during the operation is transmitted to the control unit 17 of the massage machine 10. In this manner, a voice such as "The treatment will be weakened a little" is output by the control unit 17, and the treatment of the treatment unit can be further weakened.

If the conversation is exchanged in this way while the treatment target person is treated, based on the content of the conversation exchanged between the treatment target person and the artificial intelligence unit 51, the artificial intelligence unit 51 properly determines the treatment suitable for the treatment target person at that time. In this manner, the artificial intelligence unit 51 notifies the treatment target person of the suitable treatment, and can properly adjust the operation of the massage machine 10.

### <Others>

In addition to factors for determining the treatment suitable for the treatment target person as described above, the artificial intelligence unit 51 can confirm whether or not the treatment content is suitable for the treatment course (the treatment content) selected by the treatment target person through the conversation. For example, with regard to the treatment course in which the treatment target person operates the operation device 20, the artificial intelligence unit 51 may ask the treatment target person a question such as "Are you satisfied with the usual treatment course today?" If the treatment target person answers to the question from the artificial intelligence unit 51 such as "Slightly weak" or "Slightly strong", the artificial intelligence unit 51 transmits a control signal suitable for the conversation to the control unit 17 of the massage machine 10. The control unit 17 can adjust the massage machine 10 so that desired treatment can be performed in the treatment course selected by the treatment target person.

The factors for determining the treatment suitable for the treatment target person by the artificial intelligence unit 51 can be combined with each other. Depending on conditions, the artificial intelligence unit 51 appropriately combines the factors with each other. In addition, the factors for determining the treatment suitable for the treatment target person by the artificial intelligence unit 51 as described above are merely examples. It is possible to add various factors thereto within the scope without impairing the gist of the present invention. The factors are not limited to the above-described examples.

### Summary

As described above, according to the massage system 1 including the above-described massage machine 10, based on the conversation exchanged between the treatment target person and the artificial intelligence unit 51, the artificial intelligence unit 51 can determine the treatment suitable for the health condition of the treatment target person at that time. Accordingly, the treatment target person can recognize and receive the treatment suitable for a state of his or her mind and body when using the massage machine 10.

Therefore, depending on the physical conditions and feelings which vary every day, the treatment target person can receive the treatment suitable for himself or herself at that time in the massage machine 10.

In the above-described embodiment, the massage system 1 including the three chair-type massage machines 10 has been described as an example. However, even if the massage machine 10 is not the chair-type massage machine, massage machines having mutually different types may be mixed with each other, and the number of the massage machines is not limited to the above-described embodiment.

In addition, in the above-described embodiment, the server 50 includes the artificial intelligence unit 51. However, the artificial intelligence unit 51 may be configured to be independent of the server 50, and a configuration relating to the artificial intelligence unit 51 is not limited to the above-described embodiment.

Furthermore, the above-described embodiment shows an example, the content of the conversation exchanged between the treatment target person and the artificial intelligence unit 51 may vary due to various factors. Various conversations are available as long as the gist of the present invention is not impaired. The present invention is not limited to the above-described embodiment.

### LIST OF REFERENCE NUMERALS

- 1: MASSAGE SYSTEM
- 2: WIRELESS COMMUNICATION
- 3: COMMUNICATION NETWORK
- 10: MASSAGE MACHINE (CHAIR-TYPE MASSAGE MACHINE)
- 12: BACKREST UNIT
- 15: TREATMENT UNIT (ROLLER UNIT)
- 16: STORAGE UNIT
- 17: CONTROL UNIT
- 20: OPERATION DEVICE
- 21: SCREEN
- 22: VOICE INPUT UNIT
- 23: VOICE OUTPUT UNIT
- 24: CAMERA (IMAGING UNIT)
- 30: MEASUREMENT UNIT
- 31: SPHYGMOMANOMETER
- 32: BODY COMPOSITION METER
- 40: TERMINAL
- 50: SERVER
- 51: ARTIFICIAL INTELLIGENCE UNIT
- 52: INFORMATION STORAGE UNIT

## Claims

1. A massage system including a massage machine, the massage system comprising:
a massage machine that has a treatment unit for treating a treatment target person, a control unit for controlling the treatment unit, a voice input unit, and a voice output unit; and
a server that is connected to the massage machine via a communication network,
wherein the massage machine transmits voice information of the treatment target person which is input from the voice input unit, to the server via the communication network,
wherein the server causes an artificial intelligence unit to determine treatment suitable for the treatment target person, based on the voice information of the treatment target person, and transmits the treatment to the massage machine, and
wherein the massage machine is configured to output the transmitted treatment from the voice output unit.

2. The massage system including a massage machine according to Claim 1,
wherein the massage machine includes a physical information acquisition unit that acquires physical information relating to the treatment target person,
wherein the control unit transmits the physical information of the treatment target person which is acquired by the physical information acquisition unit, to the server via the communication network, and
wherein the server is configured to determine treatment suitable for the treatment target person by causing the artificial intelligence unit to consider the physical information of the treatment target person in addition to the voice information of the treatment target person.

3. The massage system including a massage machine according to Claim 1 or 2,
wherein at least any one of the massage machine and the server includes an information storage unit that stores personal information of the treatment target person.

4. The massage system including a massage machine according to any one of Claims 1 to 3,
wherein the artificial intelligence unit extracts information relating to the treatment of the treatment target person from the voice information of the treatment target person, and
wherein the artificial intelligence unit is configured to determine the treatment suitable for the treatment target person by considering the information relating to the treatment of the treatment target person.

5. The massage system including a massage machine according to any one of Claims 1 to 4,
wherein the artificial intelligence unit determines feelings of the treatment target person, based on the voice information of the treatment target person, and is configured to determine the treatment suitable for the treatment target person by considering information of the feelings.

6. The massage system including a massage machine according to any one of Claims 1 to 5,
wherein the artificial intelligence unit is configured to determine the treatment suitable for the treatment target person by determining activity information of the treatment target person from the voice information of the treatment target person and by considering the activity information.

7. The massage system including a massage machine according to any one of Claims 1 to 6,
wherein the artificial intelligence unit determines information relating to a body of the treatment target person from the voice information of the treatment target person, and is configured to determine the treatment suitable for the treatment target person by considering the information relating to the body.

8. The massage system including a massage machine according to any one of Claims 1 to 7,
wherein the massage machine includes an imaging unit that images a face of the treatment target person, and
wherein the artificial intelligence unit performs determination based on a Visual diagnosis method from the face of the treatment target person which is imaged by the imaging unit, and is configured to determine the treatment suitable for the treatment target person by considering the determination based on the Visual diagnosis method in addition to the determination based on the voice information of the treatment target person.

9. The massage system including a massage machine according to any one of Claims 1 to 8,
wherein at least any one of the massage machine and the server causes a history storage unit to store a usage history including the treatment determined based on the voice information of the treatment target person, and
wherein the artificial intelligence unit is configured to determine the treatment suitable for the treatment target person by considering the usage history stored in the history storage unit in addition to new voice information of the treatment target person.

10. The massage system including a massage machine according to any one of Claims 1 to 9,
wherein the artificial intelligence unit asks the treatment target person a question or answers a question from the treatment target person, and
wherein in a case where the artificial intelligence unit obtains an answer to the question from the treatment target person, the artificial intelligence unit is configured to determine the treatment suitable for the treatment target person from a plurality of answers by obtaining a new answer from the treatment target person after asking a question relating to the obtained answer.

11. The massage system including a massage machine according to any one of Claims 1 to 10,
wherein the artificial intelligence unit exchanges a casual conversation with the treatment target person, and is configured to determine the treatment suitable for the treatment target person from the content of the conversation.

12. The massage system including a massage machine according to any one of Claims 1 to 11,
wherein the artificial intelligence unit confirms the treatment of the massage machine from the voice output unit while the treatment target person is treated,
wherein the artificial intelligence unit determines whether or not to change an operation of the massage machine, based on the answer of the treatment target person which is input from the voice input unit, and
wherein in a case where the artificial intelligence unit determines that the operation of the massage machine needs to be changed, the artificial intelligence unit is configured to notify the treatment target person of the determined change result from the voice output unit of the massage machine.

13. The massage system including a massage machine according to any one of Claims 1 to 12,
wherein the massage machine is a chair-type massage machine that has at least a seat unit on which the treatment target person sits, and a backrest unit that supports an upper body of the treatment target person sitting on the seat unit from behind, and
wherein the control unit is configured to adjust an angle of at least the backrest unit to a suitable angle, based on the determination of the artificial intelligence unit.
